# EUROPEAN PATENT SPECIFICATION

(11) **EP 4 600 007 B1**
(45) Date of publication and mention of the grant of the patent: **25.11.2026**
(21) Application number: 24848854.6
(22) Date of filing: 10.07.2024
(51) Int. Cl.: B29B 17/00, G01N 21/35, G01N 21/3563, G01N 29/04, B29K 67/00, B29L 7/00, B29C 33/68

(54) **METHOD FOR RECYCLING RELEASE SHEET**
VERFAHREN ZUR WIEDERVERWERTUNG EINER TRENNFOLIE
PROCÉDÉ DE RECYCLAGE DE FEUILLE ANTIADHÉSIVE

(30) Priority: 31.07.2023 JP 2023125004
(43) Date of publication of application: 13.08.2025
(73) Proprietor: NEION Film Coatings Corp., Higashiosaka-shi, Osaka 578-0935 (JP)
(72) Inventor: SHIMIZU, Mamoru, Higashiosaka-shi, Osaka 578-0935 (JP); SHIMIZU, Kanzo, Higashiosaka-shi, Osaka 578-0935 (JP); KAWABATA, Hiroki, Higashiosaka-shi, Osaka 578-0935 (JP)
(74) Representative: advotec.
(86) International application number: PCT/JP2024/024911
(87) International publication number: WO 2025/028195

(56) References cited:
- EP-A1- 3 505 880
- JP-A- 2000 177 057
- JP-A- 2002 214 136
- JP-A- 2023 079 071
- JP-A- H0 910 703
- KR-A- 20210 048 236
- "Release-Liner Waste Management - More than a Burning Issue", PACKAGE PRINT. CONVERTING,, vol. 39, no. 7, 1 July 1992 (1992-07-01), pages 25 - 27, XP009146986

## Description

### TECHNICAL FIELD

The technique disclosed herein relates to a release sheet recycling method.

### BACKGROUND ART

In recent years, the reuse and recycling of various products, from industrial products to daily necessities, have been proposed due to growing awareness of environmental protection. The collection and recycling of resin products receive particular attention following the enactment of the Plastic Resource Circulation Promotion Act. For example, the reuse of resin base materials used in a manufacturing process for laminates, including polarizing plates, has been considered (see, for example, Patent Document 1).

In recycling, it is important to collect and sort materials efficiently. Building up a recycling system for printing materials has also been considered (see, for example, Patent Document 2). Patent Document 3 discloses a method of collecting label-stock material from multiple geographical locations for recycling. At each site a plurality of containers are stored and are filled with material and are provided with an identification marker such as an RFID tag. The fill factors of the various containers are predicted using models related to the quantity of material being formed as waste during the automated application of labels to items.

### CITATION LIST

### PATENT DOCUMENT

Patent Document 1: Japanese Unexamined Patent Publication No. 2015-118388
Patent Document 2: Japanese Unexamined Patent Publication No. 2017-139008
Patent Document 3: EP 3 505 880 A1
Patent Document 4: KR 2021 0048236 A
Patent Document 5: JP 2000 177057 A

### SUMMARY OF THE INVENTION

### TECHNICAL PROBLEM

In recent years, efforts have been made not only to recycle resin materials but to establish a closed-loop recycling system, where resin materials collected from used products are recycled and used as the materials of the same products. In closed-loop recycling, the recycled resin material must maintain the quality close to that of unused material; thus, contamination of foreign matter during collection of the used material causes a bigger problem.

As a form of closed-loop recycling, new attempts are being made to collect release liners (sheets) from used labels made of adhesive layers and the release liners, recycle them into resin material, and use them again in the manufacture of release liners. Although labels come in various types and materials, a single type of release liner can be used for many labels. Thus, effective recycling is possible by collecting the release liners. However, since closed-loop recycling of release liners is a new approach, the methods for achieving this have not been fully established.

An object of the present invention is to provide a release liner recycling method capable of removing foreign matter with high accuracy while recycling release liners efficiently, and provide a release liner suitable for this method.

### SOLUTION TO THE PROBLEM

The scope of the invention is set out in the appended set of claims. A release liner (sheet) recycling method disclosed herein includes: supplying a resin sheet laminate to a user company, the resin sheet laminate including: an adhesive sheet having an adhesive layer (pressure sensitive adhesive layer); and a release liner adhering to the adhesive sheet, the release liner having a first surface on which a release layer is formed and a second surface opposite to the first surface and having a base made of a resin material; collecting a used release liner peeled from the resin sheet laminate from the user company in any form selected from a rolled sheet or a cut sheet using a collection bin; by using an analysis system including a storage unit, an irradiation unit, a detection unit, and a determination unit, storing a composition of the release liner in the storage unit; conducting a sorting inspection of a contained item in the collection bin collected from the user company; removing foreign matter determined not to be the used release liner by the sorting inspection from the contained item in the collection bin; and recycling the used release liner into a resin raw material. The conducting a sorting inspection of the contained item in the collection bin includes: detecting, by the detection unit, visible light, infrared light, or ultrasonic waves emitted from the irradiation unit to the contained item and reflected from the contained item or transmitted through the contained item; analyzing the composition of the contained item based on the visible light, the infrared light, or the ultrasonic waves detected by the detection unit; checking the result of the analysis against the composition of the release liner stored in the storage unit; and determining the rolled sheet or the cut sheet, among contained items, whose composition does not match the stored composition of the release liner is not the used release liner.

### ADVANTAGES OF THE INVENTION

The recycling method disclosed in the present specification can use a composition inspection of a release liner with visible light, infrared light, or ultrasonic waves as a sorting inspection, enabling efficient recycling of used release liners.

### BRIEF DESCRIPTION OF THE DRAWINGS

[FIG. 1] FIG. 1 is a cross-sectional view of a resin sheet laminate including an example of a release liner applied to a release liner recycling method according to a first embodiment of the present disclosure.
[FIG. 2] FIG. 2 is a plan view of the release liner shown in FIG. 1, as viewed from the surface in contact with an adhesive layer.
[FIG. 3] FIG. 3 is a cross-sectional view of a resin sheet laminate including another example of the release liner applied to the release liner recycling method according to the first embodiment.
[FIG. 4] FIG. 4 is a flowchart showing the release liner recycling method according to the first embodiment.
[FIG. 5] FIG. 5 is a block diagram showing an inspection system for use in the recycling method according to the first embodiment.
[FIG. 6] FIG. 6 is a flowchart showing the details of the steps of the sorting inspection of contained items.

### DESCRIPTION OF EMBODIMENTS

### (First Embodiment)

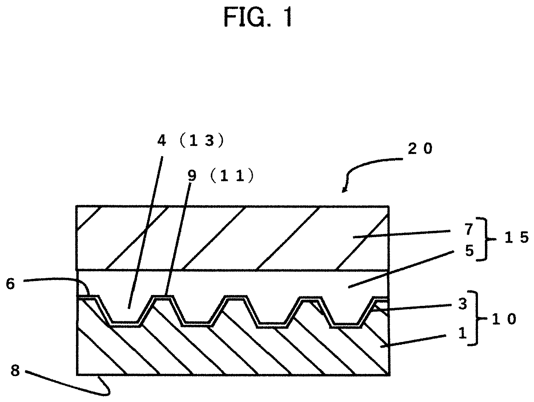

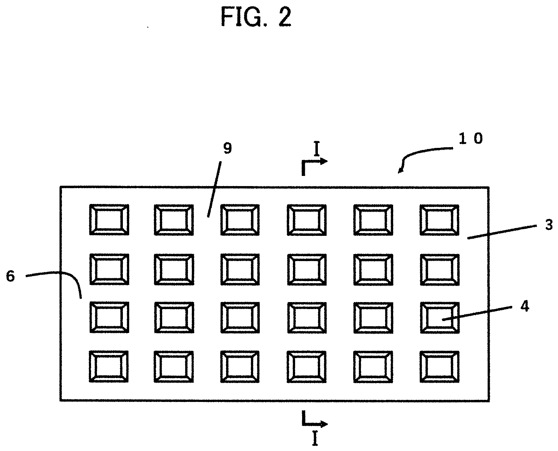

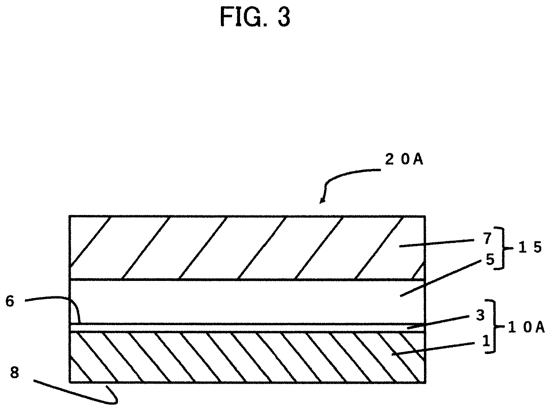

FIG. 1 is a cross-sectional view of a resin sheet laminate including a release liner. FIG. 2 is a plan view of the release liner, as viewed from the surface in contact with an adhesive layer. FIG. 3 is a cross-sectional view of another example of the release liner. These release liners 10 and 10A are applied to a recycling method according to the first embodiment.

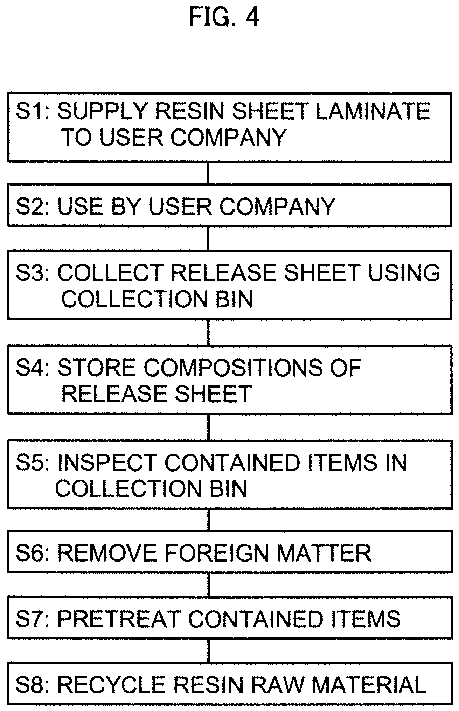

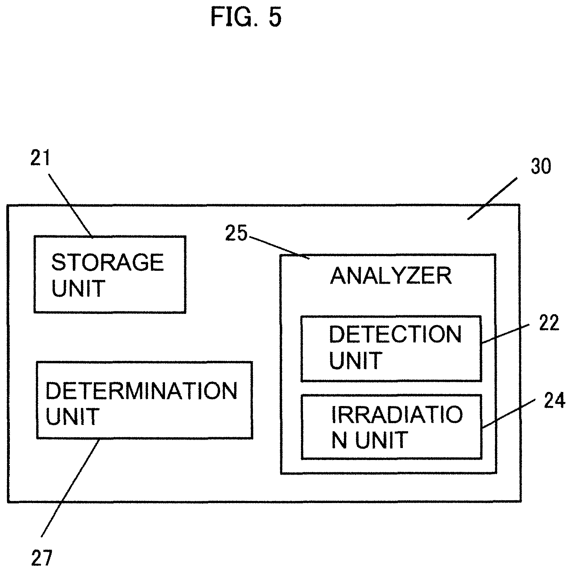

FIG. 4 is a flowchart showing the release liner recycling method according to the first embodiment. FIG. 5 is a block diagram showing an analysis system for use in the recycling method according to this embodiment.

In the recycling method of the present embodiment, the release liner 10 peeled from a resin sheet laminate 20 shown in FIG. 1 or the release liner 10A peeled from a resin sheet laminate 20A is collected. The resin sheet laminate 20 includes: an adhesive sheet 15 having a base sheet 7 and an adhesive layer 5 provided on one surface of the base sheet 7; and the release liner 10 adhering to the adhesive layer 5.

The release liner 10 has a first surface 6 on which a release layer 3 is formed and a second surface 8 opposite to the first surface 6. The release liner 10 has a base 1 made of a resin material, and a three-dimensional shape 4 is formed on at least one of the first surface 6 or the second surface 8. The "three-dimensional shape" used herein refers to a three-dimensional shape with respect to a plane, such as a recess, a projection, and recesses and projections, and may be any shape as long as it is three-dimensional. The base 1 is in the form of sheet. FIGS. 1 and 2 show an example in which the three-dimensional shape 4 is a recess depressed in a truncated pyramid shape. The first surface 6 has a pattern of recesses and projections in which recesses are repeatedly arranged at regular intervals in two-dimensional directions, and the adhesive layer 5 has a reverse pattern of recesses and projections corresponding to the pattern of the first surface 6, in which a plurality of projections 13 are defined by a grid of grooves 11. It can also be said that the pattern of the recesses and projections formed in the first surface 6 is made by a grid of ridges 9.

This configuration allows air to escape from the grooves 11 when the adhesive sheet 15 is applied to an adherend, so that the adhesive sheet 15 can adhere to the adherend without trapping air.

A release liner having a flat release surface without a three-dimensional shape, such as the release liner 10A of the resin sheet laminate 20A shown in FIG. 3, can also be recycled by the present recycling method.

The base sheet 7 may be made of at least one material selected from a resin sheet, paper, and synthetic paper, or may be a laminate of these materials. When the adhesive sheet 15 is a label or the like, at least one of the surface of the base sheet 7 in contact with the adhesive layer 5 or the surface opposite to this surface may have an easy-adhesion layer that improves adhesion with the ink.

The size of the pattern of the recesses and projections of the release liner 10 is not particularly limited. However, the following pattern is preferable because air entrapment can be effectively reduced when applying the adhesive sheet 15: a pattern in which the height of the three-dimensional shape 4 is, for example, about 5 µm or more and 100 µm or less, the length of a side of the perimeter is about 20 µm or more and 1000 µm or less, the distance between adjacent three-dimensional shapes 4 is about 20 µm or more and 1000 µm or less, and in which it is possible to form the grooves 11 as air passages in the adhesive layer 5.

The adhesive layer 5 may be made of at least one type of adhesives selected from urethane-based, acryl-based, silicone-based, polyester-based, and rubber-based adhesives. A plant-derived adhesive may be used as the adhesive, or a plant-derived tackifier or the like may be added.

The base 1 of the release liner 10, 10A only needs to be made of a recyclable resin material. The resin material is not particularly limited, but polyester resins, particularly polyethylene terephthalate (PET), are preferably used because of their high versatility and well-established recycling techniques. The base 1 may be transparent or white, but may be free from coloring with ink or the like from the viewpoint of reuse. When the base 1 is made of a porous resin material, the color is white. The porous base 1 can reduce the use amount of the resin material as compared with the case where a transparent general resin film is used. For example, KAMISHINE (trademark) from TOYOBO Co., Ltd. can be used as the base 1. From an environmental perspective, the base 1 may contain biomass polyester, including plant-derived materials.

The release layer 3 contains a release agent containing a silicone resin, a fluororesin, or the like and is formed to follow the shape of the three-dimensional shapes 4 in the example shown in FIG. 1. The thickness of the release layer 3 is about 5 µm or less in general. The second surface 8 and/or the first surface 6 of the base 1 may be provided with an antistatic layer.

The analysis system 30 shown in FIG. 5 has, for example, a storage unit 21 that can store information on the composition of the release liner 10, 10A, such as the thickness and material of each layer, an analyzer 25 that analyzes the composition of the inspection target in the state of a rolled sheet or a cut sheet, and a determination unit 27.

The analyzer 25 has an irradiation unit 24 that irradiates the inspection target with visible light, infrared light, ultrasonic waves, or the like, and a detection unit 22 that detects reflected light from the inspection target or transmitted light through the inspection target and analyzes the layer composition, thickness, and constituent materials of the inspection target. The system of the analyzer 25 is not particularly limited, but it is desirable that the analyzer 25 can detect differences in the composition of inspection targets with sufficient accuracy. When the analyzer 25 uses infrared light, the analyzer 25 may be of a dispersion type or a Fourier transform (FT-IR) type. The use of the Fourier transform type analyzer 25 allows high-speed analysis with high accuracy.
The determination unit 27 checks the information on the composition of the release liner 10, 10A read from the storage unit 21 against the composition of the inspection target analyzed by the analyzer 25. If the composition of the inspection target matches the composition of the release liner 10, 10A, the rolled sheet or the cut sheet is determined to be the release liner 10, 10A. If the composition of the inspection target does not match the composition of the release liner 10, 10A, the rolled sheet or the cut sheet is determined not to be the release liner 10, 10A. In this step, whether the inspection target is foreign matter or not is determined for each rolled sheet and each cut sheet.

At least one of the storage unit 21 or the determination unit 27 may be provided inside the analyzer 25 or may be provided outside the analyzer 25. The analyzer 25 may be a portable, handheld type, or may be a stationary type with a fixed position. The stationary analyzer 25 allows successive inspection of the inspection targets conveyed by a carrier such as a belt conveyor. For use in the successive inspection, it is preferable that the analysis by the analyzer 25 be completed within, for example, one second. The handheld analyzer 25 can be used even when the analysis requires several to several dozen seconds, and can also be used at closer positions to the inspection target, which makes it possible to detect the reflected light or the transmitted light more reliably and thus improve the accuracy of the analysis.

The release liner 10, 10A is recycled by the procedure shown in FIG. 4. First, in step S1, the resin sheet laminate 20, 20A is supplied/sold to a user company or sold to a processing company, which processes the resin sheet laminate 20, 20A and supply it to the user company. The processing company makes prints on a surface of the adhesive sheet 15 and carries out processing such as punching and half-cutting. In some cases, the user company carries out processing such as printing by themselves.

Next, in step S2, the user company supplied with the resin sheet laminate 20, 20A or its processed product peels the adhesive sheet 15 from the release liner 10, 10A and uses the adhesive sheet 15. The user company does not discard but stores and accumulates the used release liner 10, 10A after peeling.

In step S3, the used release liners 10, 10A accumulated to a predetermined amount are collected from the user company in any form selected from a rolled sheet or a cut sheet using a collection bin. The length of the rolled sheet, the size of the cut sheet, and the presence or absence of a cut portion are not particularly limited.

In step S4, the storage unit 21 stores the compositions of the release liners 10, 10A. Specifically, the storage unit 21 stores the layer compositions of the release liners 10, 10A, the material and thicknesses of each layer, and the like. This step may be performed at any time before the subsequent sorting inspection.

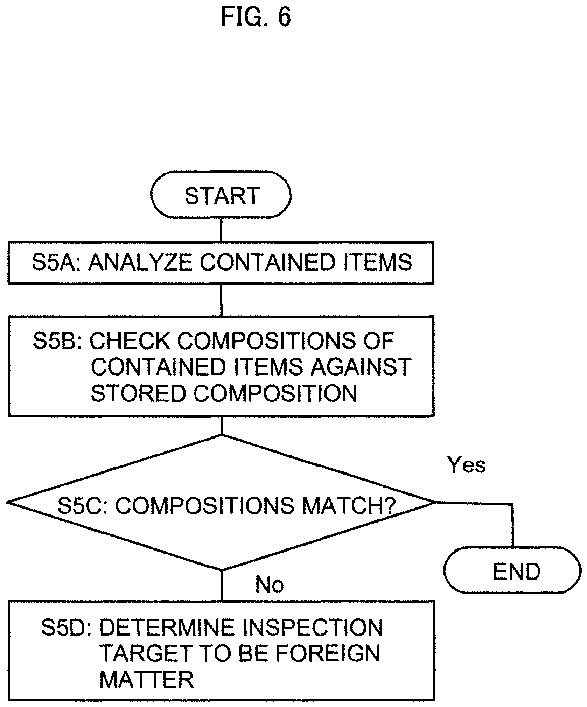

In step S5, the sorting inspection of the contained items in the collection bin is conducted. The sorting inspection S5 includes an analysis test for analyzing the compositions of the contained items by utilizing visible light, infrared light, or ultrasonic waves to determine whether the contained items are the release liners 10, 10A. The details of the analysis test are shown in FIG. 6.

In step S5A, the compositions of the contained items in the collection bin are analyzed for each rolled sheet or cut sheet in sequence. The analysis result by the analyzer 25 is sent to the determination unit 27.

Next, in steps S5B and S5C, the determination unit 27 checks the compositions of the contained items against the composition of the release liner 10, 10A stored in the storage unit 21. Among the contained items, the rolled sheet or the cut sheet whose composition does not match the stored composition of the release liner 10, 10A is determined not to be the used release liner 10, 10A by the determination unit 27. The rolled sheet or the cut sheet whose composition matches the composition of the release liner 10, 10A is determined to be the used release liner 10, 10A.

If it is determined that the inspection target is not the used release liner 10, 10A, the inspection target is determined as foreign matter in step S5D. This method allows easy detection of release liners with different shapes but the same sheet composition, such as the release liner 10 and the release liner 10A. Thus, even when multiple types of release liners need to be recycled, the release liners can be reliably sorted. On the other hand, if sorting by optical characteristics using visible light is difficult, such as when the release liner 10, 10A is white, infrared light or ultrasonic waves can be used for accurate sorting based on the sheet structure.

If the wettability of the first surface 6 of the release liner 10, 10A is smaller than that of the second surface 8 and the wettability of each surface is known, the wettability can be tested in the sorting inspection S5. The wettability can be tested by using, for example, a dyne pen having a wettability greater than that of the first surface 6 and equal to or smaller than that of the second surface 8. Suppose, as a concrete example, that the wettability of the first surface of the release liner 10, 10A is smaller than 30 dyn/cm (= 30 mN/m) and the wettability of the second surface is 40 dyn/cm (= 40 mN/m), and the ink of a dyne pen of 40 dyn/cm is applied to both surfaces of the contained item (i.e., rolled sheet or cut sheet). Then, if the ink is repelled on both surfaces after 5 seconds and if the ink is not repelled on both surfaces, the contained item can be easily determined not to be the release liner 10, 10A. Whether or not the ink is repelled can be confirmed visually or by a camera or the like connected to the analyzer.

In the wettability test, either both or only one of the wettability test or the analysis test in sub-step 5A, in which the contained items are analyzed using light or ultrasonic waves, can be performed. It is possible to select appropriately which one of the result of the wettability test and the result of the analysis test is prioritized in making the determination. For example, if the results of both the analysis test and the wettability test are consistent, the determination can be made according to the result; if the results are inconsistent, the determination can be made by prioritizing the result of the analysis test or the wettability test which is determined in advance. It can also be determined that the contained items are not the release liners 10, 10A if the test results are inconsistent. The determination unit of the analyzer can make a determination, using the result of the analysis test and the result of the wettability test.

Since the wettability test can be applied regardless of the color or transparency of the release liner 10, 10A, it is preferable to use the result of the wettability test if the release liner 10, 10A is opaque and difficult to be sorted by optical characteristics. In addition, while it is difficult for the analysis test using visible light, infrared light, or ultrasonic waves to confirm the presence or absence of the release layer 3, the wettability test easily confirms the presence or absence of the release layer 3. Therefore, even if a resin sheet with the same resin composition as the base 1 of the release liner 10, 10A but without the release layer 3 is mixed in the contained items in the collection bin, using the wettability test in combination can reduce the risk of misidentifying such a resin sheet without the release layer 3 as the used release liner 10, 10A. This can significantly improve the reliability of the sorting inspection without incurring high costs.

The wettability test may be performed either before or after the analysis test using visible light, infrared light, or ultrasonic waves. If the wettability test is conducted first, it is preferable not to omit but conduct the analysis test using visible light, infrared light, or ultrasonic waves even if the result of the wettability test shows that the contained items are not used release liners 10, 10A. Conducting both tests makes it easier to identify the type of the foreign matter detected in step S5. Thus, recording the history of the inspection results in a memory or the like makes it easier to implement measures to prevent the mixing of foreign matter. The analysis test can be omitted if the wettability test result indicates that the contained items are not used release liners 10, 10A. If the wettability test is performed after the analysis test, the wettability test can be conducted regardless of the result of the analysis test, or whether to conduct or omit the wettability test can be determined depending on the result of the analysis test.

In some cases, the resin sheet laminate 20, 20A has a configuration in which a plurality of release liners 10, 10A are connected by a connection tape. In this case, the connection tape can be sorted easily based on the difference in material between the connection tape and the release liners 10, 10A. Only a portion of the inspection target may be inspected if the inspection target is a rolled sheet.

Next, in step S6 shown in FIG. 4, foreign matter determined not to be used release liners 10, 10A in the sorting inspection is removed from the contained items in the collection bin.

In the step S7, the contained items may be pretreated as necessary. For example, the used release liners 10, 10A may be washed with warm water or alkaline water to remove the release layer 3 and/or the antistatic layer, and then cut into an appropriate size and heated and melted into pellet form. The used release liners 10, 10A may be made into pellet form without removing the release layer 3 unless there is any particular problem. As a pretreatment, surfaces of the used release liners 10, 10A may be polished. There are methods of polishing both surfaces of the used release liners 10, 10A at the same time and polishing only one surface. In the case of polishing only one surface, the first surface 6, where the release layer 3 is formed, can be identified by simply conducting the above-described wettability test using ink. This enables easy removal of the release layer 3.

In step S8, the used release liners 10, 10A are recycled into a resin raw material. The method of recycling the resin raw material is not particularly limited, and various methods can be used according to the resin composition of the release liner 10. The recycled resin raw material can be used again for the production of release liners. It is possible to produce release liners using only recycled raw material, but the recycled raw material may be mixed with unused raw materials. Considering the environmental impact, it is preferable that the base of newly produced release liners contain at least 10 mass% or more of the recycled resin raw material. The resin raw material recycled from the used release liners, that is, the resin raw material recycled in a closed-loop system, may be contained in the release liners 10, 10A. The use of the material recycled in a closed-loop system is preferred as it leads to a direct saving in petroleum resources.

In the method of the present embodiment, the adhesive sheet 15 included in the resin sheet laminate 20, 20A does not necessarily have to have the base sheet 7 and may be sandwiched between two release liners 10 or 10A without the base sheet. Alternatively, in step S1, the resin sheet laminate 20, in which the release layer 3 is applied to both surfaces of the base 1 and in which the release liner 10 is provided on only one surface of a so-called base-less adhesive sheet 15, may be sold. In this case, the resin sheet laminate 20 may be supplied as a roll that is rolled with the back surface (second surface) of the release liner 10 being in contact with the surface of the adhesive layer 5 opposite to the release liner 10.

The storage process (step S4), the inspection process (step S5), and the foreign matter removal process (step S6) shown in FIG. 4 may be successively performed after the receipt of the release liners collected from the user company in step S3. Alternatively, step S4 and step S5 may be performed as the inspection when accepting the release liners collected from the user company. In this case, acceptance may be denied when foreign matter is found in the release liners in an amount equal to or greater than the allowable limit.

According to the recycling method of the present embodiment, it is possible to detect and remove foreign matter accurately and efficiently.

### INDUSTRIAL APPLICABILITY

The release liner recycling method of the present disclosure is useful in the field of environmentally friendly adhesive sheets, such as labels.

### DESCRIPTION OF REFERENCE CHARACTERS

- 1: Base
- 3: Release Agent Layer
- 4: Three-Dimensional Shape
- 5: Adhesive Layer
- 7: Base Sheet
- 9: Ridge
- 10, 10A: Release liner
- 11: Groove
- 13: Projections
- 15: Adhesive Sheet
- 20, 20A: Resin Sheet Laminate
- 21: Storage Unit
- 22: Detection Unit
- 24: Irradiation Unit
- 25: Analyzer
- 27: Determination Unit
- 30: Analysis System

## Claims

1. A release liner recycling method, comprising:
supplying a resin sheet laminate (20, 20A) to a user company, the resin sheet laminate (20, 20A) comprising: an adhesive sheet (15) having an adhesive layer (5); and a release liner (10, 10A) adhering to the adhesive sheet (15), the release liner (10, 10A) having a first surface on which a release layer (3) is formed and a second surface opposite to the first surface and having a base (1) made of a resin material;
collecting the used release liner peeled from the resin sheet laminate (20, 20A) from the user company in any form selected from a rolled sheet or a cut sheet using a collection bin;
determining, as a determination step, whether a contained item in the collection bin collected from the user company is the used release liner by conducting a sorting inspection of the contained item;
removing foreign matter determined not to be the used release liner in the determination step from the contained item in the collection bin; and
recycling the used release liner into a resin raw material,
the determination step including an analysis test of determining whether the contained item is the used release liner by utilizing visible light, infrared light, or ultrasonic waves, using an analysis system (30) equipped with a storage unit (21), an irradiation unit (24), a detection unit (22), and a determination unit (27),
the analysis test including:
a sub-step of detecting, by the detection unit (22), the visible light, the infrared light, or the ultrasonic waves emitted from the irradiation unit (24) to the contained item and reflected from the contained item or transmitted through the contained item; and
a sub-step of determining whether the contained item is the used release liner by checking in the determination unit (27) whether a detection result by the detection unit (22) matches a composition of the release liner (10, 10A) stored in the storage unit (21) in advance, wherein a wettability of the release liner (10, 10A) is smaller on the first surface than on the second surface,
the determination step includes a wettability test,
the wettability test includes: an application step of applying ink having a wettability greater than the wettability of the first surface and equal to or smaller than the wettability of the second surface to both surfaces of the contained item; a detection step of detecting whether the applied ink is repelled; and a determination step of determining whether the contained item is the used release liner, based on a detection result in the detection step, and
if the contained item is determined not to be the used release liner in the wettability test, the determination step determines that the contained item is not the used release liner regardless of the determination in the analysis test.

2. The release liner recycling method of claim 1, wherein
the release liner (10, 10A) includes a release liner having a flat shape and a release liner with recesses and projections on the first surface.

3. The recycling method according to claim 1, wherein
the analysis system (30) includes a handheld analyzer (25) including the irradiation unit (24) and the detection unit (22).

4. The recycling method of claim 1, wherein
the base (1) of the release liner (10, 10A) is made of a polyester resin.

5. The recycling method of any one of claims 1 to 4, wherein
the base (1) of the release liner (10, 10A) contains 10 mass% or more of recycled resin raw material.

## Patentansprüche

1. Trennpapierrecyclingverfahren, folgende Schritte umfassend:
Liefern eines Harzfolienlaminats (20, 20A) an ein Verwenderunternehmen, wobei das Harzfolienlaminat (20, 20A) Folgendes umfasst: eine Klebefolie (15) mit einer Klebeschicht (5);
und ein Trennpapier (10, 10A), das an der Klebefolie (15) klebt, wobei das Trennpapier (10, 10A) eine erste Oberfläche, auf der eine Trennschicht (3) ausgebildet ist, und eine zweite Oberfläche, die der ersten Oberfläche gegenüberliegt und eine Grundfläche (1) aus einem Harzmaterial aufweist, aufweist;
Sammeln des von dem Harzfolienlaminat (20, 20A) abgezogenen benutzten Trennpapiers von dem Verwenderunternehmen in einer beliebigen Form, welche ausgewählt ist aus einer aufgerollten Folie oder einer zugeschnittenen Folie, unter Verwendung eines Sammelbehälters;
als ein Bestimmungsschritt Bestimmen, ob ein von dem Verwenderunternehmen gesammeltes enthaltenes Objekt in dem Sammelbehälter das benutzte Trennpapier ist, indem eine Sortierprüfung des enthaltenen Objekts durchgeführt wird;
Entfernen von Fremdkörpern, bei denen in dem Bestimmungsschritt bestimmt wird, dass sie nicht das benutzte Trennpapier sind, aus dem enthaltenen Objekt in dem Sammelbehälter; und
Recyceln des benutzten Trennpapiers zu einem Harzrohstoff, wobei der Bestimmungsschritt einen Analysetest umfasst, bei dem unter Verwendung eines Analysesystems (30), das mit einer Speichereinheit (21), einer Bestrahlungseinheit (24), einer Erfassungseinheit (22) und einer Bestimmungseinheit (27) ausgestattet ist, bestimmt wird, ob das enthaltene Objekt das benutzte Trennpapier ist, indem sichtbares Licht, Infrarotlicht oder Ultraschallwellen eingesetzt werden,
wobei der Analysetest folgende Schritte umfasst:
einen Unterschritt des Erfassens des sichtbaren Lichts, des Infrarotlichts oder der Ultraschallwellen, das/die von der Bestrahlungseinheit (24) zu dem enthaltenen Objekt emittiert wird/werden und von dem enthaltenen Objekt reflektiert wird/werden oder durch das enthaltene Objekt hindurch gesendet wird/werden, durch die Erfassungseinheit (22) ; und
einen Unterschritt des Bestimmens, ob das enthaltene Objekt das benutzte Trennpapier ist, indem in der Bestimmungseinheit (27) überprüft wird, ob ein Erfassungsergebnis von der Erfassungseinheit (22) mit einer in der Speichereinheit (21) im Voraus gespeicherten Zusammensetzung des Trennpapiers (10, 10A) übereinstimmt, wobei eine Benetzungsfähigkeit des Trennpapiers (10, 10A) auf der ersten Oberfläche kleiner ist als auf der zweiten Oberfläche, wobei der Bestimmungsschritt einen Benetzungsfähigkeitstest umfasst,
wobei der Benetzungsfähigkeitstest folgende Schritte umfasst: einen Auftragungsschritt des Auftragens von Tinte, die eine Benetzungsfähigkeit aufweist, welche größer als die Benetzungsfähigkeit der ersten Oberfläche ist und gleich der oder kleiner als die Benetzungsfähigkeit der zweiten Oberfläche ist, auf beide Oberflächen des enthaltenen Objekts; einen Erfassungsschritt des Erfassens, ob die aufgetragene Tinte abgestoßen wird; und
einen Bestimmungsschritt des Bestimmens, ob das enthaltene Objekt das benutzte Trennpapier ist, auf der Basis eines Erfassungsergebnisses in dem Erfassungsschritt, und
wobei, wenn in dem Benetzungsfähigkeitstest bestimmt wird, dass das enthaltene Objekt nicht das benutzte Trennpapier ist, der Bestimmungsschritt bestimmt, dass das enthaltenen Objekt, unabhängig von der Bestimmung in dem Analysetest, nicht das benutzte Trennpapier ist.

2. Trennpapierrecyclingverfahren nach Anspruch 1, wobei
das Trennpapier (10, 10A) ein Trennpapier mit einer flachen Form und ein Trennpapier mit Ausnehmungen und Vorsprüngen auf der ersten Oberfläche umfasst.

3. Trennpapierrecyclingverfahren nach Anspruch 1, wobei
das Analysesystem (30) ein tragbares Analysegerät (25) umfasst, das die Bestrahlungseinheit (24) und die Erfassungseinheit (22) umfasst.

4. Trennpapierrecyclingverfahren nach Anspruch 1, wobei die Grundfläche (1) des Trennpapiers (10, 10A) aus einem Polyesterharz ausgebildet ist.

5. Trennpapierrecyclingverfahren nach einem der Ansprüche 1 bis 4, wobei
die Grundfläche (1) des Trennpapiers (10, 10A) 10 Masse-% oder mehr an recyceltem Harzrohstoff umfasst.

## Revendications

1. Procédé de recyclage de doublures de dégagement, comprenant les étapes suivantes :
fournir un stratifié (20, 20A) de feuilles de résine à une entreprise utilisatrice, le stratifié (20, 20A) de feuilles de résine comprenant :
une feuille adhésive (15) ayant une couche adhésive (5) ; et une doublure de dégagement (10, 10A) qui adhère à la feuille adhésive (15), la doublure de dégagement (10, 10A) ayant une première surface sur laquelle une couche de dégagement (3) est formée et une deuxième surface opposée à la première surface et ayant une base (1) faite d'un matériau de résine ;
collecter la doublure de dégagement usagée retirée du stratifié (20, 20A) de feuilles de résine de l'entreprise utilisatrice sous n'importe quelle forme sélectionnée parmi une feuille enroulée ou une feuille découpée en utilisant un récipient collecteur ;
déterminer, comme étape de détermination, si un objet contenu dans le récipient collecteur collecté de l'entreprise utilisatrice est la doublure de dégagement usagée en effectuant une inspection de triage de l'objet contenu ;
enlever des corps étrangers, dont il est déterminé dans l'étape de détermination qu'ils ne sont pas la doublure de dégagement usagée, de l'objet contenu dans le récipient collecteur ; et
recycler la doublure de dégagement usagée en une matière première de résine,
l'étape de détermination comprenant un essai d'analyse destiné à déterminer, à l'aide d'un système d'analyse (30) équipé d'une unité de stockage (21), d'une unité d'irradiation (24), d'une unité de détection (22) et d'une unité de détermination (27), si l'objet contenu est la doublure de dégagement usagée en utilisant de la lumière visible, de la lumière infrarouge ou des ondes ultrasoniques, l'essai d'analyse comprenant les étapes suivantes :
une sous-étape consistant à détecter, par l'unité de détection (22), la lumière visible, la lumière infrarouge ou les ondes ultrasoniques émises à partir de l'unité d'irradiation (24) vers l'objet contenu et réfléchies par l'objet contenu ou transmises à travers l'objet contenu ; et
une sous-étape consistant à déterminer si l'objet contenu est la doublure de dégagement usagée en vérifiant dans l'unité de détermination (27) si un résultat de détection par l'unité de détection (22) correspond à une composition de la doublure de dégagement (10, 10A) stockée dans l'unité de stockage (21) d'avance,
dans lequel une mouillabilité de la doublure de dégagement (10, 10A) est plus faible sur la première surface que sur la deuxième surface,
dans lequel l'étape de détermination comprend un essai de mouillabilité,
dans lequel l'essai de mouillabilité comprend les étapes suivantes :
une étape d'application consistant à appliquer de l'encre, qui a une mouillabilité supérieure à la mouillabilité de la première surface et égale ou inférieure à la mouillabilité de la deuxième surface, sur les deux surfaces de l'objet contenu ; une étape de détection consistant à détecter si l'encre appliquée est repoussée ; et une étape de détermination consistant à déterminer si l'objet contenu est la doublure de dégagement usagée sur la base d'un résultat de détection dans l'étape de détection, et
dans lequel, s'il est déterminé dans l'essai de mouillabilité que l'objet contenu n'est pas la doublure de dégagement usagée, l'étape de détermination détermine que l'objet contenu n'est pas la doublure de dégagement usagée indépendamment de la détermination dans l'essai d'analyse.

2. Procédé de recyclage de doublures de dégagement selon la revendication 1, dans lequel
la doublure de dégagement (10, 10A) comprend une doublure de dégagement ayant une forme plate et une doublure de dégagement avec des évidements et des saillies sur la première surface.

3. Procédé de recyclage de doublures de dégagement selon la revendication 1, dans lequel
le système d'analyse (30) comprend un analyseur portatif (25) comprenant l'unité d'irradiation (24) et l'unité de détection (22).

4. Procédé de recyclage de doublures de dégagement selon la revendication 1, dans lequel
la base (1) de la doublure de dégagement (10, 10A) est faite d'une résine polyester.

5. Procédé de recyclage de doublures de dégagement selon l'une quelconque des revendications 1 à 4, dans lequel la base (1) de la doublure (10, 10A) contient 10 % en poids ou plus d'une matière première recyclée de résine.
